(19) Europäisches Patentamt
European Patent Office
Office européen des brevets

(11) Veröffentlichungsnummer: **0 014 456**
**B1**

(12) # EUROPÄISCHE PATENTSCHRIFT

(45) Veröffentlichungstag der Patentschrift: **23.06.82**

(51) Int. Cl.[3]: **C 07 D 239/10**

(21) Anmeldenummer: **80100559.6**

(22) Anmeldetag: **04.02.80**

(54) **Hexahydropyrimidyl-(4)-äther und Verfahren zu ihrer Herstellung.**

(30) Priorität: **10.02.79 DE 2905072**

(43) Veröffentlichungstag der Anmeldung: **20.08.80 Patentblatt 80/17**

(45) Bekanntmachung des Hinweises auf die Patenterteilung: **23.06.82 Patentblatt 82/25**

(84) Benannte Vertragsstaaten: **BE CH DE FR GB NL**

(56) Entgegenhaltungen: **DE-A-1 770 089**

(73) Patentinhaber: **BASF Aktiengesellschaft, Carl-Bosch-Strasse 38, D-6700 Ludwigshafen (DE)**

(72) Erfinder: **Petersen, Harro, Dr., Kalmitstrasse 23, D-6710 Frankenthal (DE)**
Erfinder: **Fischer, Kurt, Mundenheimer Strasse 168, D-6700 Ludwigshafen (DE)**
Erfinder: **Segnitz, Adolph, Dr., Im Roehrich 46, D-6702 Bad Duerkheim 1 (DE)**
Erfinder: **Zaunbrecher, Horst, Freiligrathstrasse 1, D-6520 Worms (DE)**

EP 0 014 456 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Hexahydropyrimidyl-(4)-äther und Verfahren zu ihrer Herstellung

Die Erfindung betrifft neue Hexahydropyrimidyl-(4)-äther und ein Verfahren zur Herstellung von Hexahydropyrimidyl-(4)-äthern durch Umsetzung von Hexahydropyrimidinen mit aromatischen Alkoholen bei Temperaturen über 110°C.

Für die Herstellung von 2-Oxo(thiono)-hexahydropyrimidyl-4-äthern ist ein Verfahren bekannt, bei dem Harnstoffe mit Aldehyden in Gegenwart einer Säure bei Temperaturen unterhalb 110°C umgesetzt werden (Synthesis 1973, Seiten 262 und 263; DE-PS 1 231 247). Nach den Angaben muß mit einem hohen Überschuß der Alkohole umgesetzt werden, um für die technischen Verwendungsgebiete befriedigende Ausbeuten zu erhalten. Setzt man säureempfindliche Alkohole, z. B. oxäthylierte Carbonsäuren, ungesättigte Carbonsäuren mit Hydroxylgruppen, Polyalkohole, Polyätherdiole, ein, liefert dieses Herstellungsverfahren keine wesentliche Menge an Endstoff. Die Neutralisation der Säure und Abtrennung der dabei entstehenden Salze sind weitere Erschwernisse bei der Aufarbeitung.

Die deutsche Offenlegungsschrift 1 770 089 lehrt ein Verfahren zur Herstellung von fluorierten 4-Alkoxy-propylenharnstoffen durch Umsetzung von 4-Alkoxy(Hydroxy)-propylenharnstoffen mit fluorierten aliphatischen Alkoholen in Gegenwart einer nicht oxidierenden Säure. Es wird angegeben, daß die Umsetzung in der Regel bei einer Temperatur zwischen 20 und 100°C, vorzugsweise zwischen 40 und 80°C, durchgeführt wird.

Aus den Monatsheften für Chemie, Band 96 (1965), Seiten 1950 bis 1966 (insbesondere Seite 1951, 1956 und 1962) ist bekannt, daß Hexahydropyrimidyl-(4)-alkyläther mit Phenolen in Gegenwart von Säuren, am siedenden Wasserbad erhitzt, z. B. in der folgenden Weise reagieren:

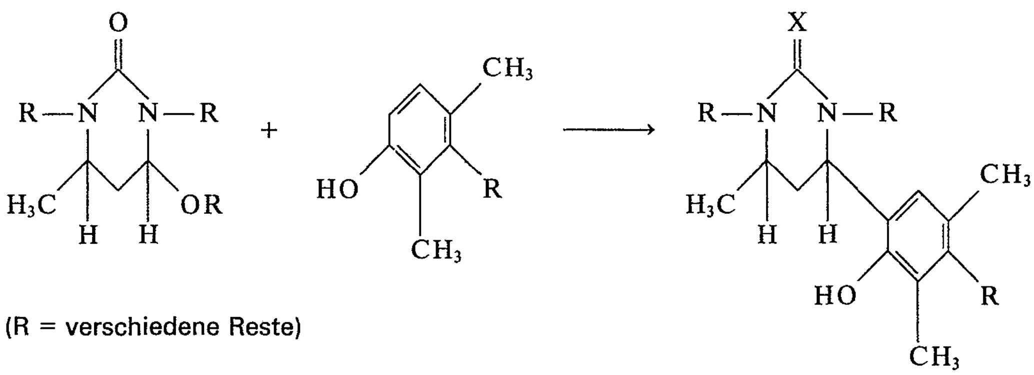

(R = verschiedene Reste)

In allen Fällen bildet sich kein Phenoläther.

Es wurde nun gefunden, daß man Hexahydropyrimidyl-(4)-äther der Formel

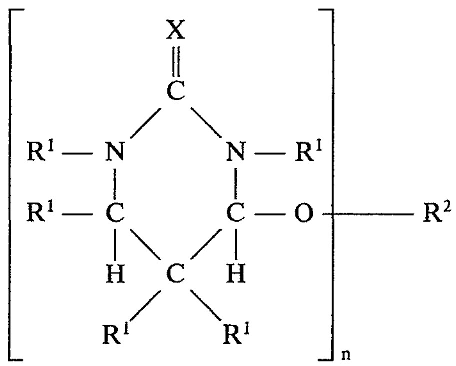

(I)

worin die einzelnen Reste $R^1$ und $R^2$ gleich oder verschieden sein können und jeweils einen Phenylrest oder Naphthylrest, der jeweils noch durch 1 bis 3 Alkylgruppen mit 1 bis 12 Kohlenstoffatomen, Aralkylgruppen oder Alkylarylgruppen mit 7 bis 12 Kohlenstoffatomen, Phenylgruppen, Carbalkoxygruppen mit 2 bis 6 Kohlenstoffatomen, Cyangruppen, Dialkylaminogruppen mit 2 bis 8 Kohlenstoffatomen, substituiert sein kann, bedeuten, darüber hinaus auch die Reste $R^1$ jeweils ein Wasserstoffatom, einen Aralkylrest mit 7 bis 12 Kohlenstoffatomen oder einen Alkylrest mit 1 bis 7 Kohlenstoffatomen bezeichnen können, $R^2$ auch für einen gegebenenfalls durch eine Phenylengruppe substituierten Phenylenrest oder Naphthylenrest oder durch eine Phenylengruppe substituierten Phenylrest oder Naphthylrest, der jeweils noch durch 1 bis 3 Alkylgruppen mit 1 bis 12 Kohlenstoffatomen, Aralkylgruppen oder Alkylarylgruppen mit 7 bis 12 Kohlenstoffatomen,

Phenylgruppen, Carbalkoxygruppen mit 2 bis 6 Kohlenstoffatomen, Cyangruppen, Dialkylaminogruppen mit 2 bis 8 Kohlenstoffatomen, substituiert sein kann, stehen kann, n eine ganze Zahl von 1 bis 3, bezeichnet, X ein Sauerstoffatom oder ein Schwefelatom bedeutet, durch Umsetzung von 4-Hydroxyhexahydropyrimidinen oder seinen Äthern mit Alkoholen vorteilhaft erhält, wenn man Hexahydropyrimidine der Formel

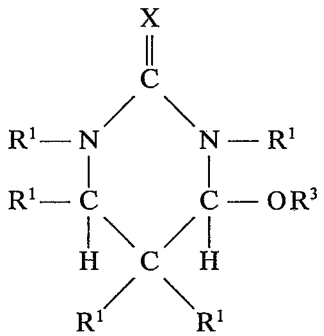

(II)

worin $R^1$ und X die vorgenannte Bedeutung besitzen und $R^3$ ein Wasserstoffatom oder eine Alkylgruppe mit 1 bis 3 Kohlenstoffatomen bezeichnet, mit Phenolen der Formel

$$[HO]_n-R^2 \qquad (III)$$

worin $R^2$ und n die vorgenannten Bedeutungen besitzen, bei einer Temperatur zwischen 110°C und 180°C umsetzt.

Weiterhin wurden die neuen Hexahydropyrimidyl-(4)-äther der Formel

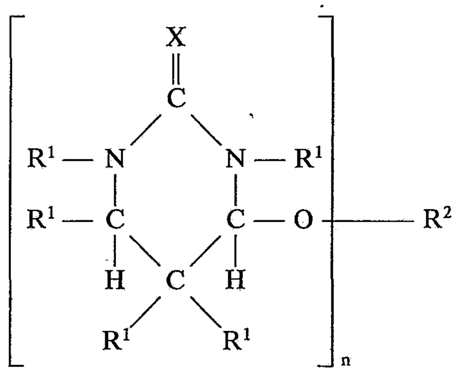

(I)

worin $R^1$, $R^2$, n und X die vorgenannte Bedeutung besitzen, gefunden.

Die Umsetzung kann für den Fall der Verwendung von Naphthol und von 2-Oxo-4-hydroxy-5,5-dimethyl-6-isopropylhexahydropyrimidin durch die folgenden Formeln wiedergegeben werden:

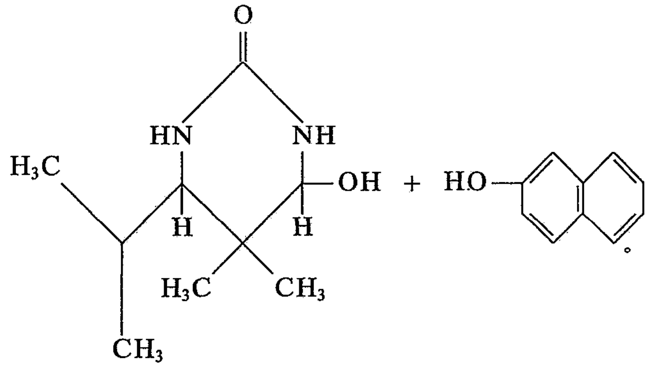

Fortsetzung

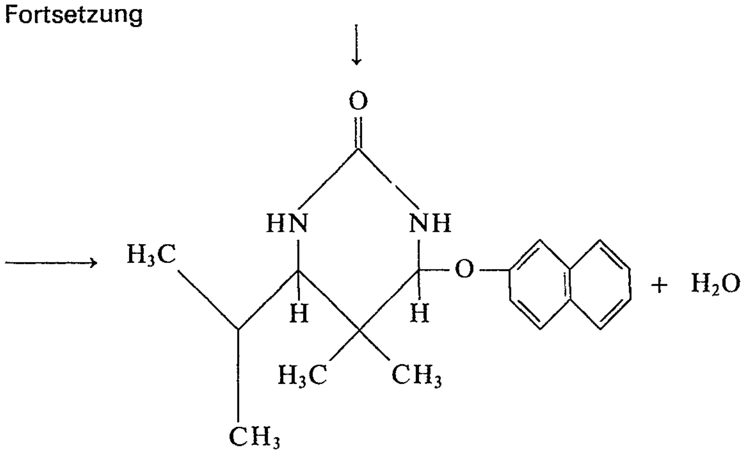

Im Hinblick auf den Stand der Technik liefert das Verfahren nach der Erfindung auf einfachem und wirtschaftlichem Wege eine große Zahl von bisher nicht beschriebenen Hexahydropyrimidyl-4-äthern in guter Ausbeute und Reinheit. Man braucht nicht in Gegenwart von Säure umzusetzen, die Neutralisation und die Abtrennung von Salzen entfallen. Zersetzungsreaktionen und Nebenreaktionen der Ausgangs- und Endprodukte werden vermieden. Ein Überschuß der Alkoholkomponente ist nicht erforderlich; eine gute Raum-Zeit-Ausbeute wird so erhalten. Alle diese vorteilhaften Ergebnisse sind im Hinblick auf den Stand der Technik überraschend. Man hätte insbesondere angesichts der höheren Reaktionstemperatur eine weit höhere Zersetzung des Reaktionsgemisches und die Bildung heterogener Gemische an Zersetzungs- und Nebenprodukten erwarten müssen.

Die Ausgangsstoffe II und III können in stöchiometrischer Menge oder im Überschuß jeder Komponente zur anderen, vorzugsweise in einem Verhältnis von 0,6 bis 1, insbesondere 0,9 bis 1 Mol Ausgangsstoff II je Hydroxygruppe und Mol Ausgangsstoff III, umgesetzt werden. Als Phenole III kommen Mono-, Di- und Triphenole in Frage. Je nach dem entsprechenden Molverhältnis des Ausgangsstoffes II zum Ausgangsstoff III erhält man in diesem Falle Mono-, Di- und Trihexahydropyrimidyl-(4)-äther bzw. bei einem Unterschuß, entsprechend den vorgenannten Mengenverhältnissen, an Ausgangsstoff II, Mono-hydroxy-poly-hexahydropyrimidyl-(4)-äther bis Polyhydroxy-mono-hexahydropyrimidyl-(4)-äther. Bevorzugte Ausgangsstoffe II und III und dementsprechend bevorzugte Endstoffe I sind solche, in deren Formeln die einzelnen Reste $R^1$ und $R^2$ gleich oder verschieden sein können und jeweils einen Phenylrest oder Naphthylrest, der jeweils noch durch 3, vorzugsweise 2 Alkylgruppen und insbesondere eine Alkylgruppe mit 1 bis 12, insbesondere 1 bis 4 Kohlenstoffatomen, substituiert sein kann, bedeuten, $R^2$ auch für einen gegebenenfalls durch eine Phenylengruppe substituierten Phenylenrest oder Naphthylenrest oder durch eine Phenylengruppe substituierten Phenylrest oder Naphthylrest, der jeweils noch durch 2 Alkylgruppen oder insbesondere 1 Alkylgruppe mit 1 bis 12, insbesondere 1 bis 4 Kohlenstoffatomen substituiert sein kann, stehen kann, n 3, vorteilhafter 2 und insbesondere 1, bezeichnet, X ein Sauerstoffatom oder ein Schwefelatom bedeutet, $R^3$ ein Wasserstoffatom oder eine Alkylgruppe mit 1 bis 3 Kohlenstoffatomen bezeichnet. Die vorgenannten Reste und Ringe können noch durch unter den Reaktionsbedingungen inerte Gruppen, z. B. Alkylgruppen mit 1 bis 4 Kohlenstoffatomen, substituiert sein.

So sind z. B. folgende Hexahydropyrimidine als Ausgangsstoffe II geeignet: 4-Hydroxy-2-oxo-hexahydropyrimidin, 5,5-Dimethyl-, 5,5-Diäthyl-, 5,5-Di-n-propyl-, 5,5-Diisopropyl-, 5,5-Di-n-butyl-, 5,5-Di-sek.-butyl-, 5,5-Diisobutyl-, 5,5-Di-tert.-butyl-, 5-Phenyl-5-methyl-, 5-Benzyl-5-methyl-, 5-Cyclohexyl-5-methyl-, 5-Methyl-5-isopropyl-4-hydroxy-2-oxo-hexahydropyrimidin; entsprechende in 5-Stellung unsubstituierte, einfach oder zweifach durch vorgenannte Gruppen substituierte, in 1-, 3- und/oder 6-Stellung durch die Methyl-, Äthyl-, Hydroxyäthyl-, n-Propyl-, Isopropyl-, n-Butyl-, Isobutyl-, sek.-Butyl-, tert.-Butyl-, Benzyl-, Phenyl-gruppe substituierte 4-Hydroxy-2-oxo-hexahydropyrimidine; homologe 4-Methoxy-, 4-Äthoxy-, 4-n-Propoxy-, 4-Isopropoxy-2-oxo-hexahydropyrimidine; entsprechende, in vorgenannten Stellungen unsubstituierte und/oder substituierte 2-Thiono-hexahydropyrimidine.

Bevorzugte Ausgangsstoffe II sind:

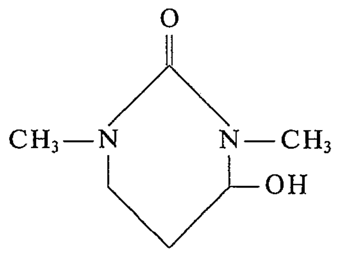

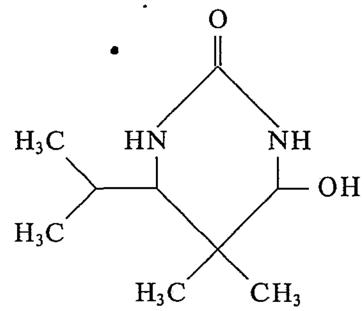

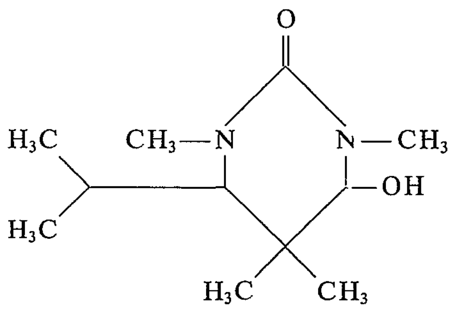

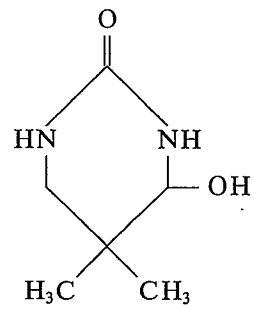

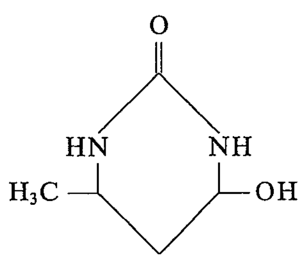

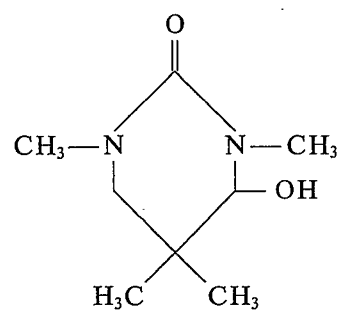

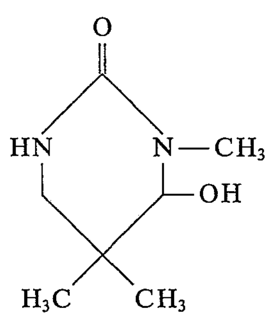

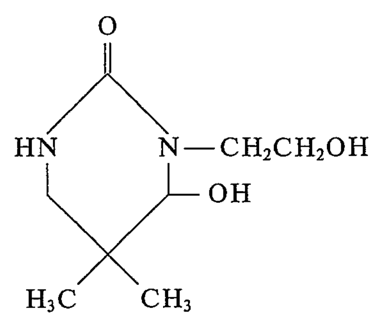

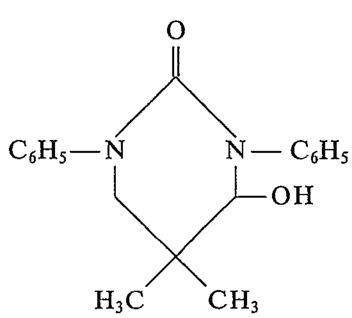

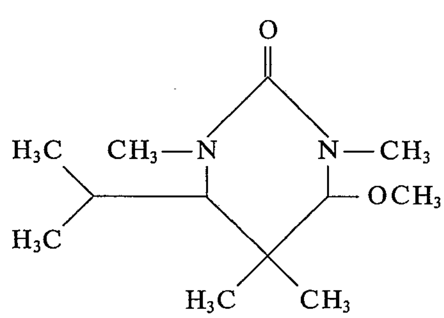

Fortsetzung

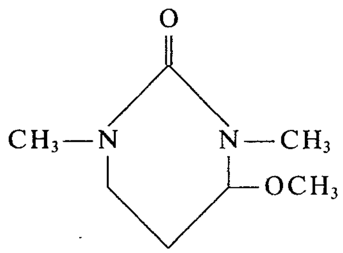

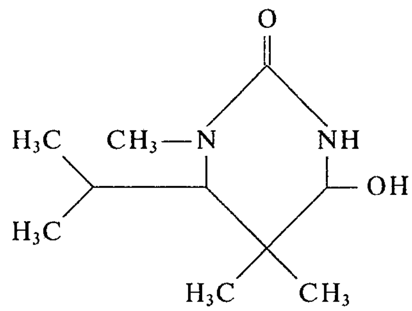

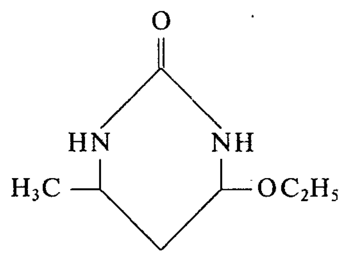

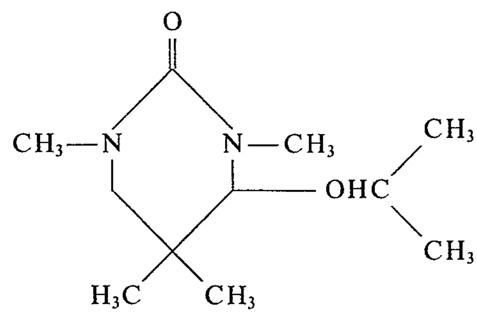

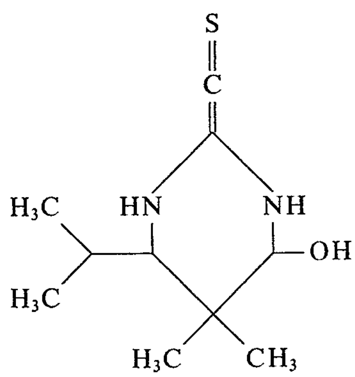

Es kommen z. B. als Phenole III in Betracht: Phenol; in 2-Stellung, 3-Stellung oder 4-Stellung einfach oder in 2,3-Stellung, 2,4-Stellung, 2,5-Stellung, 2,6-Stellung, 3,4-Stellung oder 3,5-Stellung zweifach oder in 2,4,6-Stellung, 2,3,4-Stellung, 3,4,5-Stellung, 2,4,5-Stellung, 2,3,6-Stellung, 2,3,5-Stellung dreifach gleich oder unterschiedlich durch die Methyl-, Äthyl-, Propyl-, Isopropyl-, Butyl-, Isobutyl-, sek.-Butyl-, tert.-Butyl-, Pentyl-, Hexyl-, Heptyl-, Octyl-, Nonyl-, o-Methylphenyl-, m-Methylphenyl-, p-Methylphenyl-, o-Äthylphenyl, m-Äthylphenyl-, p-Äthylphenyl-, Phenyl-, Carbmethoxy-, Carbäthoxy-, Cyan-, Dimethylamino-, Diäthylamino-, Benzyl-gruppe substituiertes Phenol; entsprechend unsubstituiertes oder einfach, zweifach oder dreifach durch vorgenannte Gruppen gleich oder unterschiedlich substituiertes $\alpha$-Naphthol oder $\beta$-Naphthol; analoge unsubstituierte oder substituierte Brenzkatechine, Resorcine, Hydrochinone, Pyrogallole, Phloroglucine, 1,2,4-Trihydroxybenzole, 1,2,5-Trihydroxybenzole, 1,2-Dihydroxynaphthole, 2,3-Dihydroxynaphthole, 4,4′-Dihydroxydiphenyle.

Bevorzugte Ausgangsstoffe III sind beispielsweise:

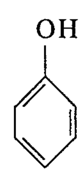

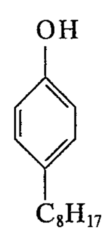

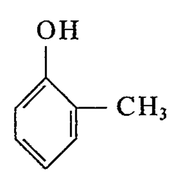

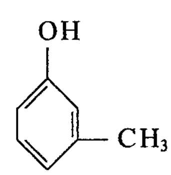

Fortsetzung

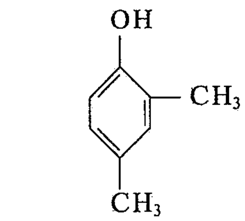 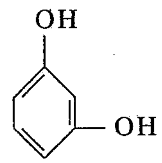 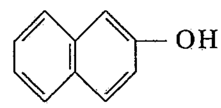 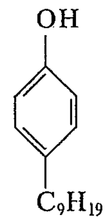

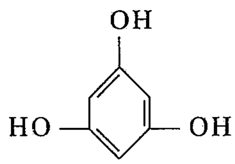 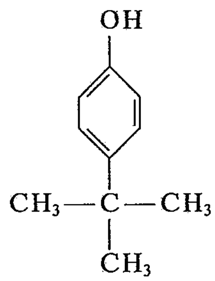

Die Umsetzung wird bei einer Temperatur zwischen 110 und 180°C, vorteilhaft zwischen 120 und 170°C, vorzugsweise von 121 bis 160°C, insbesondere von 125 bis 155°C, drucklos oder unter Druck, kontinuierlich oder diskontinuierlich durchgeführt. Man kann ohne Lösungsmittel umsetzen; zweckmäßig verwendet man aber unter den Reaktionsbedingungen inerte Lösungsmittel, insbesondere mit Siedepunkten über 110°C. Bei Flüssigkeiten mit Siedepunkten unter 110°C muß die Reaktion in geschlossenen Gefäßen unter Druck durchgeführt werden. Als Lösungsmittel kommen z. B. in Frage: aromatische Kohlenwasserstoffe, z. B. Toluol, Äthylbenzol, o-, m-, p-Xylol, Isopropylbenzol, Methylnaphthalin; Dimethylformamid; und entsprechende Gemische. Bevorzugte Lösungsmittel sind Toluol und Xylole.

Es ist nicht erforderlich, daß die Ausgangsstoffe und Endstoffe in diesen Lösungsmitteln löslich sind. Die Unlöslichkeit der Endstoffe ermöglicht eine besonders leichte Aufarbeitung. Ferner muß das gebildete Reaktionswasser nicht unbedingt abgeführt werden. Es ist jedoch vorteilhaft, das Wasser durch azeotrope Destillation abzutrennen. Zweckmäßig verwendet man das Lösungsmittel in einer Menge von 100 bis 10 000 Gewichtsprozent, vorzugsweise von 400 bis 1000 Gewichtsprozent, bezogen auf Ausgangsstoff II. Die Umsetzung kann vorteilhaft in Abwesenheit von Lösungsmitteln durchgeführt werden, wenn eine oder beide Ausgangskomponenten oder der Endstoff flüssig oder bei der Reaktionstemperatur schmelzbar sind.

Die Reaktion wird schon aus Gründen des einfacheren bzw. wirtschaftlicheren Betriebs zweckmäßig ohne Zusatz von Säure durchgeführt. Gegebenenfalls kann man aber Säure, z. B. in Gestalt von Schwefelsäure, zweckmäßig in katalytischen Mengen, bevorzugt in Mengen von 0,005 bis 0,01 Äquivalenten Säuren je Ausgangsstoff II, oder auch Basen, z. B. in Gestalt von Natronlauge, zweckmäßig in katalytischen Mengen, bevorzugt in Mengen von 0,005 bis 0,01 Äquivalenten Base je Ausgangsstoff II, zusetzen.

Die Reaktion kann wie folgt durchgeführt werden: Ein Gemisch der Ausgangsstoffe II und III und zweckmäßig Lösungsmittel wird während 0,25 bis 3 Stunden, insbesondere 0,25 bis eine Stunde, bei der Reaktionstemperatur gehalten. Dann wird der Endstoff aus dem Gemisch in üblicher Weise, z. B. durch Kristallisation und Filtration oder Destillation, abgetrennt.

Die nach dem Verfahren der Erfindung herstellbaren Hexahydropyrimidyl-(4)-äther sind wertvolle Ausgangsstoffe für die Herstellung von Farbstoffen, Schädlingsbekämpfungsmitteln und Hilfsmitteln in der Textilchemie, Lackharzchemie, Holzwerkstoffindustrie. Ihre N-Methylol- und N-Alkoxymethylverbindungen sind als reaktive Vernetzer in der Textilchemie, Lackharzchemie, Holzwerkstoffindustrie vorteilhaft verwendbar.

Die in den Beispielen genannten Teile bedeuten Gewichtsteile.

Beispiel 1

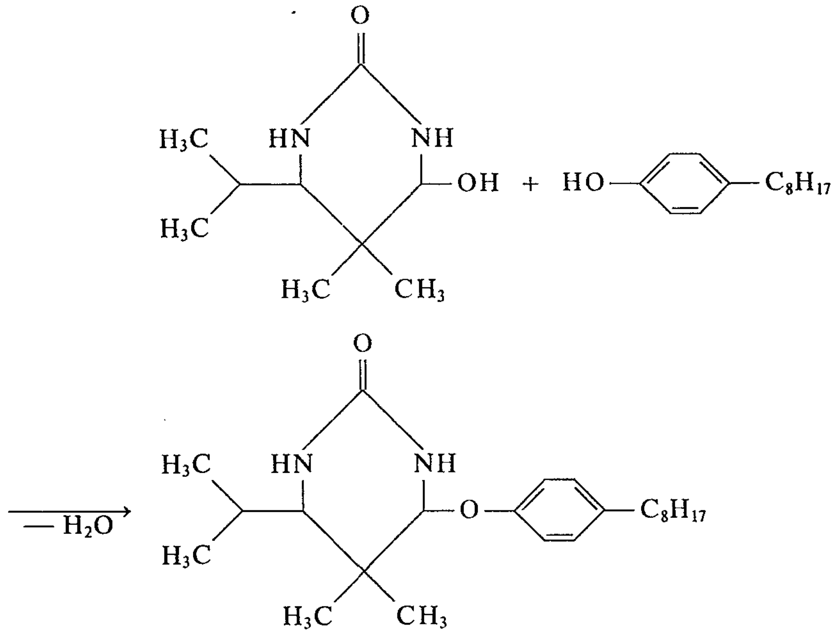


In einer Rührapparatur mit Rührer, Wasserabscheider, Rückflußkühler und Heizung werden 186 Teile 2-Oxo-4-hydroxy-5,5-dimethyl-6-isopropyl-hexahydropyrimidin zusammen mit 206 Teilen p-Octylphenol und 500 Teilen Xylol eine Stunde bei Rückflußtemperatur (130 bis 140°C) erhitzt. Das entstehende Reaktionswasser wird aus dem Reaktionsgemisch durch azeotrope Destillation über den Wasserabscheider abgetrennt. Es werden 18 Teile Wasser abgetrennt. Nach Abdestillieren des Xylols werden 371 Teile 2-Oxo-5,5-dimethyl-6-isopropyl-hexahydropyrimidyl-(4)-(p-octylphenyl)-äther erhalten. Das entspricht einer Ausbeute von 99,2% der Theorie. Fp. 145° C.

Beispiel 2

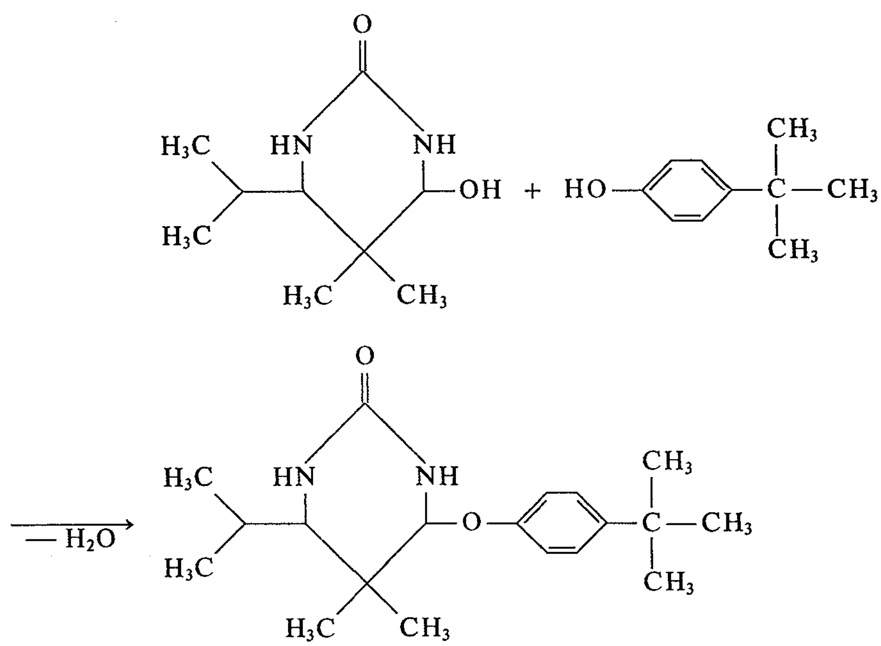


In einer in Beispiel 1 beschriebenen Rührapparatur werden 186 Teile 2-Oxo-4-hydroxy-5,5-dimethyl-6-isopropyl-hexahydropyrimidin und 150 Teile p-tertiär-butylphenol in 500 Teilen Xylol auf Rückflußtemperatur erhitzt. Bei 111 bis 140°C wird das dabei entstehende Reaktionswasser über den Wasserabscheider innerhalb von 90 Minuten abgetrennt. Es werden 18 Teile Wasser ausgekreist. Anschließend wird Xylol unter vermindertem Druck abdestilliert. Es werden 301 Teile 2-Oxo-5,5-dimethyl-6-isopropyl-hexahydropyrimidyl-(4)-(p-tertiär-butylphenyl)-äther erhalten. Das entspricht einer Ausbeute von 95% der Theorie. Fp. 65° C.

Beispiel 3

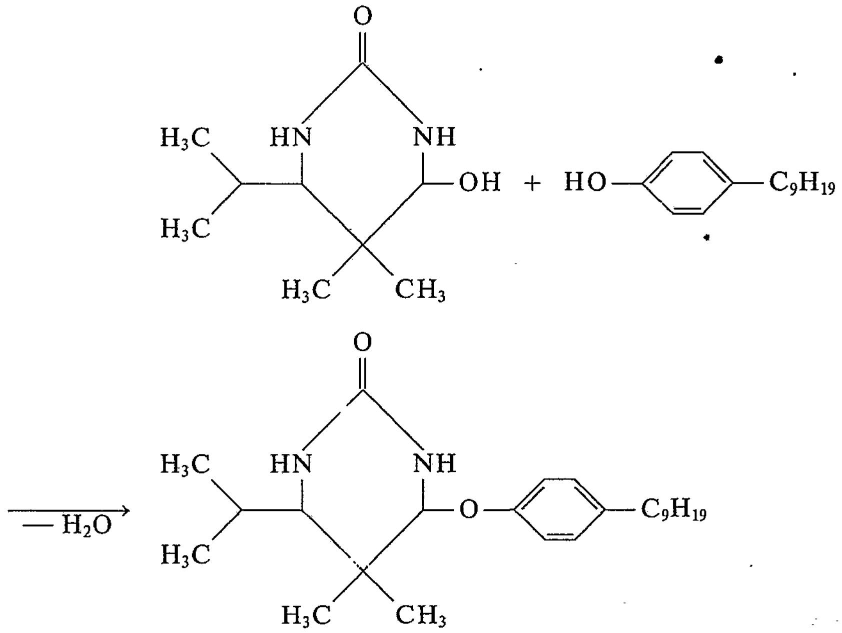


186 Teile 2-Oxo-4-hydroxy-5,5-dimethyl-6-isopropyl-hexahydropyrimidin werden zusammen mit 220 Teilen p-Nonylphenol und 500 Teilen Xylol in einer in Beispiel 1 beschriebenen Rührapparatur durch Erhitzen auf Rückflußtemperatur umgesetzt. Die Rückflußtemperatur steigt im Verlauf von 2 Stunden von 111 bis 142°C, wobei insgesamt 17,5 Teile Wasser über den Wasserabscheider abgetrennt werden. Nach Abdestillieren des Xylols unter vermindertem Druck werden 380 Teile 2-Oxo-5,5-dimethyl-6-isopropyl-hexahydropyrimidyl-(4)-(p-nonylphenyl)-äther erhalten. Das entspricht einer Ausbeute von 98% der Theorie. Fp. 55°C.

Beispiel 4

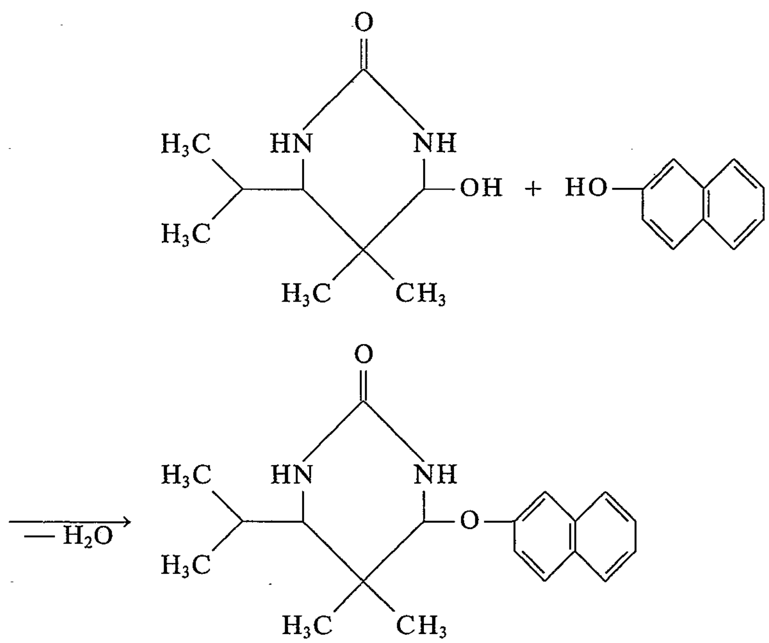


In einer in Beispiel 1 beschriebenen Rührapparatur werden 186 Teile 2-Oxo-4-hydroxy-5,5-dimethyl-6-isopropyl-hexahydropyrimidin mit 144 Teilen β-Naphthol in 500 Teilen Xylol unter Rühren auf Rückflußtemperatur erhitzt, wobei die Einsatzstoffe in Lösung gehen. Bei einer Rückflußtemperatur von 118 bis 140°C wird das Reaktionswasser im Verlauf von 2 Stunden ausgekreist, wobei 18 Teile Wasser abgetrennt werden. Anschließend wird Xylol unter vermindertem Druck abdestilliert. Es

werden 312 Teile 2-Oxo-5,5-dimethyl-6-isopropyl-hexahydropyrimidyl-(4)-(β-naphthyl)-äther erhalten. Das entspricht einer Ausbeute von 97,5% der Theorie. Fp. 211°C.

Beispiel 5

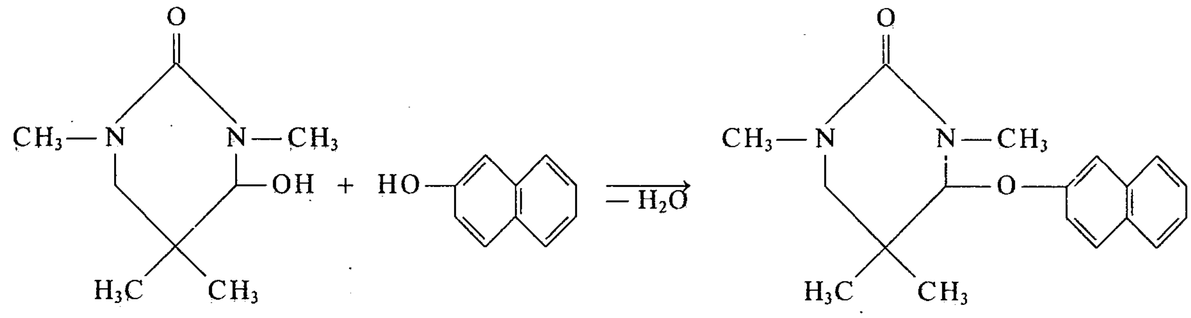


Analog Beispiel 1 werden 172 Teile 2-Oxo-4-hydroxy-1,3,5,5-tetramethyl-hexahydropyrimidin mit 144 Teilen β-Naphthol und 500 Teilen Xylol auf Rückflußtemperatur erhitzt. Die Rückflußtemperatur steigt im Verlauf von einer Stunde von 126 auf 142°C an, wobei insgesamt 17 Teile Wasser ausgekreist werden. Bereits während der Reaktionszeit fällt der Endstoff aus der heißen xylolischen Lösung aus. Nach dem Abkühlen wird der kristalline Endstoff abgesaugt und getrocknet. Es werden 266 Teile 2-Oxo-1,3,5,5-tetramethyl-hexahydropyrimidyl-(4)-(naphthyl)-äther erhalten. Das entspricht einer Ausbeute von 89% der Theorie. Fp. 250°C.

Beispiel 6

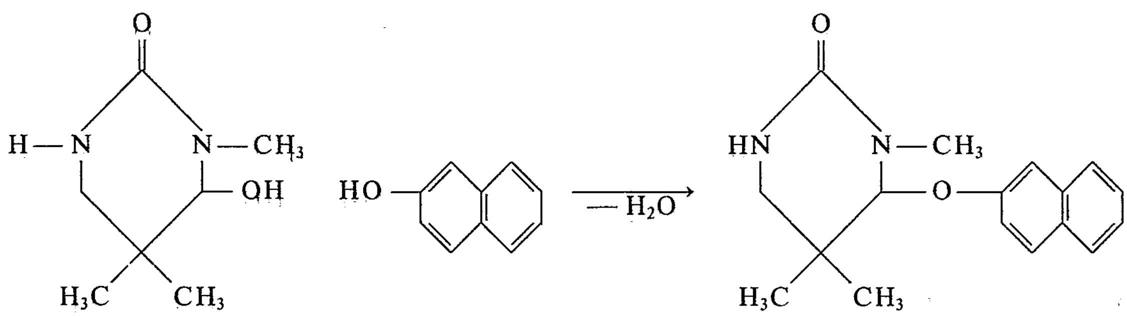


158 Teile 2-Oxo-4-hydroxy-3,5,5-trimethyl-hexahydropyrimidin und 144 Teile β-Naphthol werden in 500 Teilen Xylol unter Erwärmen auf Rückflußtemperatur in einer in Beispiel 1 beschriebenen Apparatur umgesetzt. Im Verlauf von 2 Stunden werden bei 130 bis 140°C 16,8 – Teile Wasser ausgekreist. Nach Abkühlen auf Raumtemperatur kristallisiert der Endstoff aus. Er wird nach kurzem Stehen abgesaugt und getrocknet, wobei 256 Teile 2-Oxo-3,5,5-trimethyl-hexahydropyrimidyl-(4)-(naphthyl)-äther erhalten werden. Das entspricht einer Ausbeute von 90% der Theorie. Fp. 200°C (unter Zersetzung).

Beispiel 7

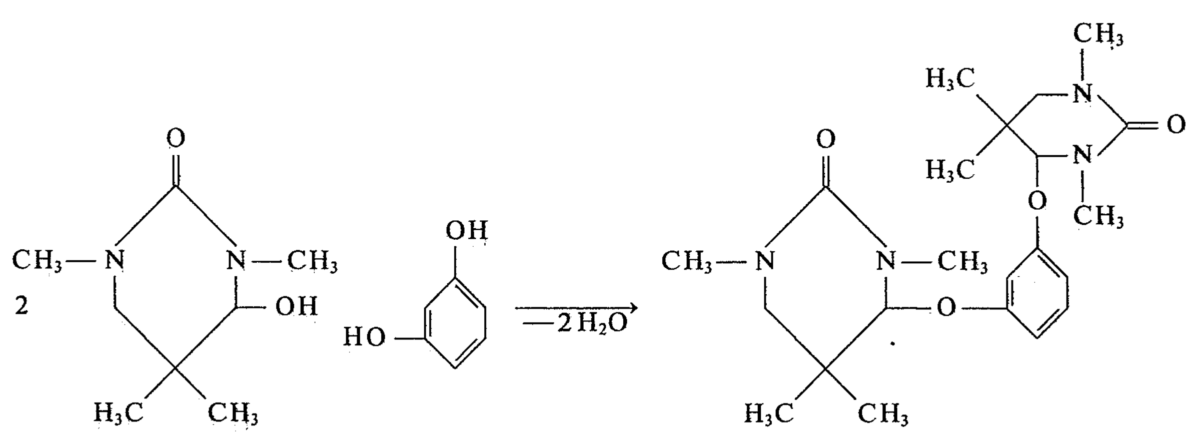

In einer in Beispiel 1 beschriebenen Rührapparatur werden 172 Teile 2-Oxo-4-hydroxy-1,3,5,5-tetramethyl-hexahydropyrimidin mit 55 Teilen Resorcin in 500 Teilen Xylol auf Rückflußtemperatur erhitzt. Die Rückflußtemperatur steigt im Verlauf von 2 Stunden von 118 auf 132° C an, wobei 17,5 Teile Wasser ausgekreist werden. Bereits während der Reaktionszeit fällt der Endstoff in kristalliner Form aus. Nach Abkühlen auf Raumtemperatur wird nach kurzem Stehen der Endstoff abfiltriert und getrocknet. Es werden 203 Teile Di-(2'-oxo-1',3',5',5'-tetramethyl-hexahydropyrimidyl-(4')-äther des Resorcins erhalten. Das entspricht einer Ausbeute von 97% der Theorie. Fp. 200° C (unter Zersetzung).

Beispiel 8 (Verwendung)

Auf ein genadeltes Vlies, bestehend aus Polyesterfasern, werden 30 Teile/$m^2$ eines nach Beispiel 1 hergestellten Harzpulvers gestreut, im Infrarotfeld bei 200° C gesintert und das Vlies anschließend kalandert. Man erhält auf diese Weise ein Vlies, das auf der einen Seite mit der Harzschicht verfestigt ist. Der textile Charakter der Warenoberseite bleibt erhalten. Das nachträglich durch Erwärmen verformbare Vlies ist im Brennverhalten im Vergleich zu mit bekannten Harzpulvern, z. B. Melamin-Formaldehydharzen, ausgerüsteten Vliesen, resistenter. Es wird vorzugsweise als Wandverspannungsmaterial verwendet.

**Patentansprüche**

1. Verfahren zur Herstellung von Hexahydropyrimidyl-(4)-äthern der Formel

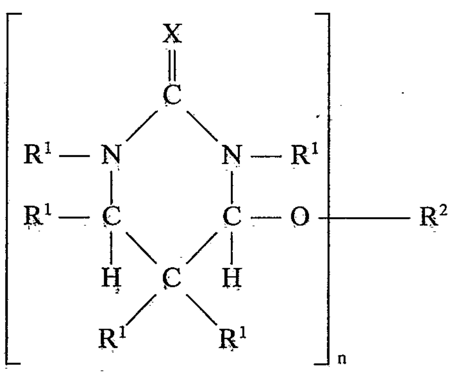

(I)

worin die einzelnen Reste $R^1$ und $R^2$ gleich oder verschieden sein können und jeweils einen Phenylrest oder Naphthylrest, der jeweils noch durch 1 bis 3 Alkylgruppen mit 1 bis 12 Kohlenstoffatomen, Aralkylgruppen oder Alkylarylgruppen mit 7 bis 12 Kohlenstoffatomen, Phenylgruppen, Carbalkoxygruppen mit 2 bis 6 Kohlenstoffatomen, Cyangruppen, Dialkylaminogruppen mit 2 bis 8 Kohlenstoffatomen, substituiert sein kann, bedeuten, darüber hinaus auch die Reste $R^1$ jeweils ein Wasserstoffatom, einen Aralkylrest mit 7 bis 12 Kohlenstoffatomen oder einen Alkylrest mit 1 bis 7 Kohlenstoffatomen bezeichnen können, $R^2$ auch für einen gegebenenfalls durch eine Phenylengruppe substituierten Phenylenrest oder Naphthylenrest oder durch eine Phenylengruppe substituierten Phenylrest oder Naphthylrest, der jeweils noch durch 1 bis 3 Alkylgruppen mit 1 bis 12 Kohlenstoffatomen, Aralkylgruppen oder Alkylarylgruppen mit 7 bis 12 Kohlenstoffatomen, Phenylgruppen, Carbalkoxygruppen mit 2 bis 6 Kohlenstoffatomen, Cyangruppen, Dialkylaminogruppen mit 2 bis 8 Kohlenstoffatomen, substituiert sein kann, stehen kann, eine ganze Zahl von 1 bis 3, bezeichnet, X ein Sauerstoffatom oder ein Schwefelatom bedeutet, durch Umsetzung von 4-Hydroxyhexahydropyrimidinen oder seinen Äthern mit Alkoholen, dadurch gekennzeichnet, daß man Hexahydropyrimidine der Formel

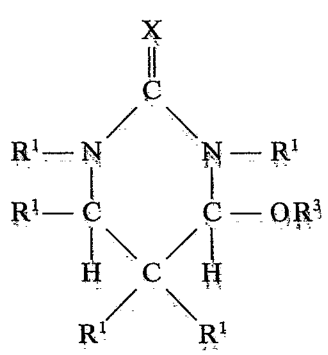

(II)

worin $R^1$ und X die vorgenannte Bedeutung besitzen und $R^3$ ein Wasserstoffatom oder eine Alkylgruppe mit 1 bis 3 Kohlenstoffatomen bezeichnet, mit Phenolen der Formel

$$[HO]_n - R^2 \qquad (III)$$

worin $R^2$ und n die vorgenannten Bedeutungen besitzen, bei einer Temperatur zwischen 110°C und 180°C umsetzt.

2. Hexahydropyrimidyl-(4)-äther der Formel

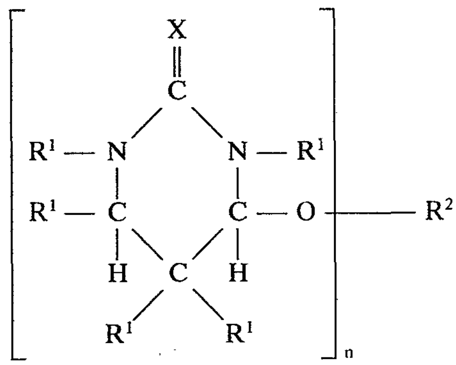

(I)

worin $R^1$, $R^2$, n und X die in Anspruch 1 genannte Bedeutung besitzen.

## Claims

1. A process for the production of hexahydropyrimid-4-yl ethers of the formula

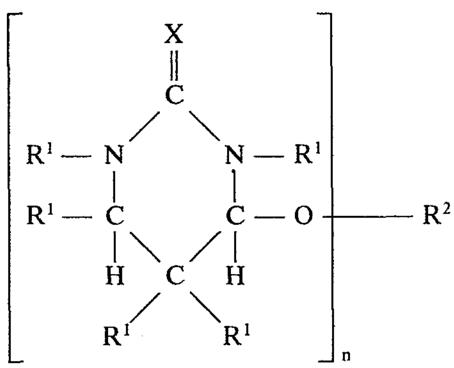

(I)

where the individual radicals $R^1$ and $R^2$ may be identical or different and each denotes phenyl or naphthyl optionally substituted by 1 to 3 alkyls of 1 to 12 carbon atoms, aralkyls or alkylaryls of 7 to 12 carbon atoms, phenyls, carbalkoxy groups of 2 to 6 carbon atoms, cyano groups, or dialkylamino groups of 2 to 8 carbon atoms, and in addition the radicals $R^1$ may each denote hydrogen, an aralkyl of 7 to 12 carbon atoms or alkyl of 1 to 7 carbon atoms, and $R^2$ may denote a phenylene or naphthylene radical optionally substituted by a phenylene group or a phenylene-substituted phenyl or naphthyl optionally substituted by 1 to 3 alkyls of 1 to 12 carbon atoms, aralkyls or alkylaryls of 7 to 12 carbon atoms, phenyls, carbalkoxy groups of 2 to 6 carbon atoms, cyano groups, or dialkylamino groups of 2 to 8 carbon atoms, n is an integer from 1 to 3, and X is oxygen or sulphur, by reacting 4-hydroxy-hexahydropyrimidines or ethers there of with alcohols, characterized in that hexahydropyrimidines of the formula

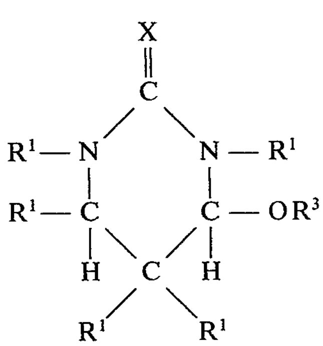

(II)

where $R^1$ and X have the above meanings and $R^3$ is hydrogen or alkyl of 1 to 3 carbon atoms, are reacted at a temperature of from 110° to 180°C with phenols of the formula

$$[HO]_n - R^2 \qquad (III)$$

where $R^2$ and n have the above meanings.

2. Hexahydropyrimid-4-yl ethers of the formula

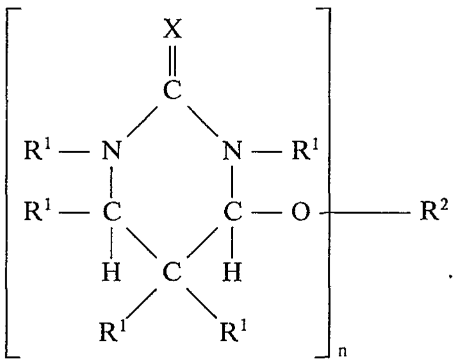

(I)

where $R^1$, $R^2$, n and X have the above meanings.

**Revendications**

1. Procédé de préparation d'hexahydro-pyrimidinyl-(4)-éthers de la formule

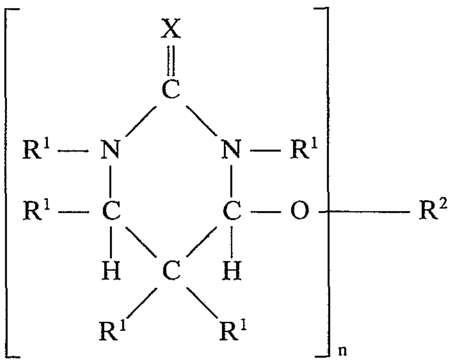

(I)

dans laquelle les divers restes $R^1$ et $R^2$, qui peuvent être identiques ou différents, désignent chacun un groupe phényle ou naphthyle pouvant être substitué par 1 à 3 radicaux alcoyle en $C_1$ à $C_{12}$, groupes aralcoyle ou alcoyl-aryle en $C_7$ à $C_{12}$, groupes phényle, groupes carbalcoxy en $C_2$ à $C_6$, groupes cyano ou groupes dialcoyl-amino en $C_2$ à $C_8$, chaque reste $R^1$ pouvant en outre représenter un atome d'hydrogène, un groupe aralcoyle en $C_7$ à $C_{12}$ ou un radical alcoyle en $C_1$ à $C_7$ et $R^2$ pouvant encore représenter un groupe phénylène ou naphthylène éventuellement substitué par un groupe phénylène ou un groupe phényle ou naphtyle substitué par un groupe phénylène et pouvant porter comme autres substituants 1à 3 radicaux alcoyle en $C_1$ à $C_{12}$, groupes aralcoyle ou alcoyl-aryle en $C_7$ à $C_{12}$, groupes phényle, groupes carbalcoxy en $C_2$ à $C_6$, groupes cyano ou groupes dialcoyl-amino en $C_2$ à $C_8$, n étant un nombre entier valant de 1 à 3 et X désignant un atome d'oxygène ou de soufre, par réaction de 4-hydroxy-hexahydro-pyrimidines ou de leurs éthers avec des alcools, caractérisé en ce que l'on fait réagir des hexahydropyrimidines de la formule

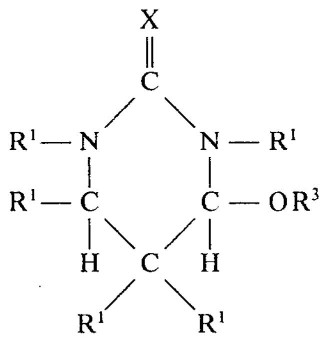

(II)

dans laquelle $R^1$ et X possèdent la signification définie plus haut et $R^3$ désigne un atome d'hydrogene ou un radical alcoyle en $C_1$ à $C_3$, à une température entre 110°C et 180°C avec des phénols de la formule

$$[HO]_n\!-\!R^2 \qquad (III)$$

dans laquelle $R^2$ et n possèdent les significations définies plus haut.

2. Hexahydro-pyrimidinyl-(4)-éthers de la formule

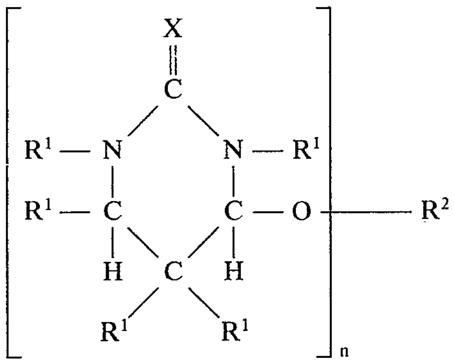

(I)

dans laquelle $R^1$, $R^2$, n et X possèdent la signification définie ci-dessus.